# FASCICULE DE BREVET EUROPEEN

(11) **EP 1 502 619 B1**
(45) Date de publication et mention de la délivrance du brevet: **26.03.2008**
(21) Numéro de dépôt: 04103500.7
(22) Date de dépôt: 22.07.2004
(51) Int. Cl.: A61M 16/00

(54) **Dispositif de fourniture de gaz respiratoire en pression ou en volume**
Vorrichtung zur Lieferung eines bestimmten Druckes oder Volumens von Beatmungsgas
Device for supplying a specific pressure or volume of breathing gas

(30) Priorité: 29.07.2003 FR 0309347
(43) Date de publication de la demande: 02.02.2005
(73) Titulaire: Airox, 64000 Pau (FR)
(72) Inventeur: Andrieux, Claude, 64800, Baudreix (FR)
(74) Mandataire: Thébault, Jean-Louis

(56) Documents cités:
- EP-A- 0 862 922
- EP-A- 1 243 282
- WO-A-02/26305
- DE-A- 19 714 644
- US-A1- 2002 005 197
- US-A1- 2002 014 239
- US-A1- 2002 053 345

## Description

La présente invention concerne un dispositif de fourniture de gaz respiratoire.

Les dispositifs de fourniture de gaz respiratoire sont utilisés notamment dans les traitements d'affections respiratoires de patients adultes ou d'enfants. Le traitement thérapeutique de telles affections consistant à insuffler au patient de l'air à deux pressions positives par rapport à l'ambiante est connu depuis de nombreuses années.

Les dispositifs de fourniture de gaz respiratoires sont aptes à fournir une quantité d'air, additionnée éventuellement d'oxygène, soit sous forme d'un volume respirable quantifié soit sous la forme d'une première pression déterminée dite pression d'inspiration et une seconde pression déterminée dite pression d'expiration.

Le document WO 00/24447 concerne un dispositif de fourniture de gaz respiratoire pourvu d'un compresseur délivrant un débit de gaz sous pression à un patient au travers d'un accumulateur/silencieux aval et d'une vanne de régulation de débit. Selon ce document le surplus de gaz non nécessaire est renvoyé à un filtre/accumulateur amont au travers d'une vanne bipasse afin de limiter les pertes de gaz respiratoire.

Le document FR 2 663 547 A1 pour sa part concerne un appareil de fourniture en continu de surpression de gaz respiratoire, cet appareil utilisant une turbine haute vitesse fournissant un gaz sous une pression déterminée constante régulée par une prise de mesure de pression au niveau du masque respiratoire du patient. Cet appareil est passif en ce que la pression est maintenue en continu, les phases d'expiration étant possibles grâce à une fuite calibrée au niveau du masque respiratoire du patient.

Le document US-A-2002/005197 prévoit lui aussi l'utilisation d'une turbine haute vitesse qui est accélérée et décélérée selon les phases du cycle patient. Ce document décrit un dispositif de fourniture de gaz respiratoire à un patient selon le préambule de la revendication indépendante 1. Par contre ce document ne prévoit pas de pilotage d'une valve d'expiration par une électrovanne proportionnelle.

Le document FR 2 822 384 A1 au nom de la demanderesse concerne un ventilateur pulmonaire mixte pourvu d'un générateur de flux, de moyens de circulation permettant de réinjecter le flux en amont du générateur lors d'une phase d'expiration d'un patient, les moyens de circulation comprenant notamment une vanne de régulation comportant deux clapets actionnés par le même actionneur permettant plusieurs modes de régulation et une optimisation des flux en fonction des phases d'inspiration et d'expiration. Selon ce document, la vitesse de rotation du générateur est maintenue constante, la pression dans le circuit patient étant contrôlée par les deux clapets qui permettent un retour de flux au générateur notamment lors des phases d'expiration.

Le document EP-A-0 862 922 prévoit pour sa part de commander une mise à l'air libre de la sortie du générateur et un circuit d'expiration par une même électrovanne recevant soit une pression issue du générateur soit la pression du générateur modulée par une fuite calibrée.

Il reste que les circuits et systèmes nécessaires pour fournir et réguler des niveaux de pression et/ou quantités volumiques de gaz respiratoire sont complexes et onéreux, la commande des clapets et vannes étant délicate.

La présente invention vise à proposer un dispositif de fourniture de gaz respiratoire perfectionné en ce que son fonctionnement utilise un asservissement de la turbine pour les phases inspiratoire et expiratoire et n'utilise pas de système de retour de gaz vers l'amont de la turbine, simplifiant ainsi le système, tout en permettant un fonctionnement avec circuit étanche au niveau du masque respiratoire ou du dispositif d'intubation du patient lors des phases d'inspiration et fuite lors des phases d'expirations.

Pour ce faire la présente invention concerne un dispositif de fourniture de gaz respiratoire à un patient selon la Revendication 1.

Des modes de réalisation particuliers de l'invention sont définis par les Revendications dépendantes 2 à 11.

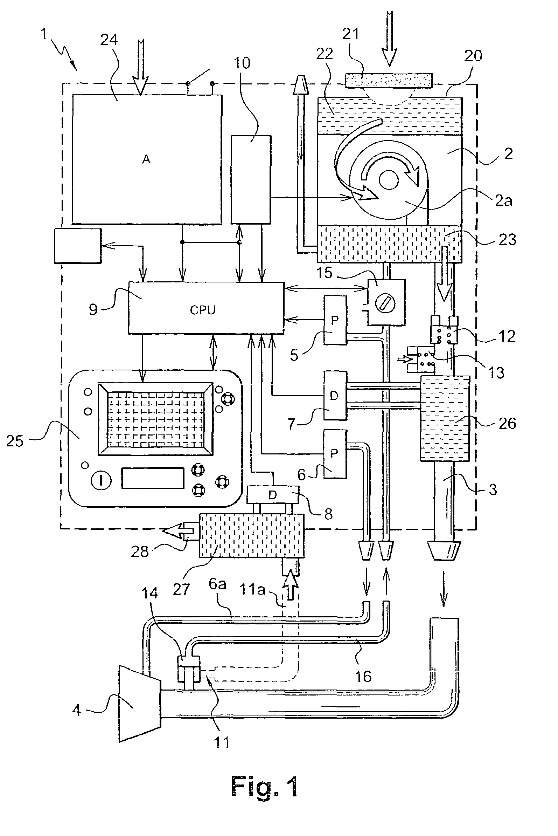

D'autres avantages de l'invention apparaîtront à la lecture de la description qui va suivre d'un exemple de réalisation non limitatif de l'invention en référence avec la figure 1 qui représente une vue schématique d'un dispositif de fourniture de gaz respiratoire selon l'invention.

Le dispositif 1 de fourniture de gaz respiratoire ou dispositif de ventilation selon la figure 1 comporte un générateur 2 de flux gazeux muni d'une turbine 2a à faible inertie et vitesse de consigne élevée. Ce générateur de flux gazeux est disposé dans une enceinte 20, précédé d'un filtre 21 pour l'entrée de l'air ambiant, de moyens d'insonorisation amont 22 et aval 23. La turbine 2a est par exemple une turbine avec vitesse de rotation maximale de l'ordre de 50 000 tours/min, apte à fournir une pression de 70 mbar au dessus de l'ambiante et un débit de l'ordre de 200 l/min.

Le générateur de flux d'air, comporte ainsi une micro-turbine à faible inertie, entraînée par un moteur électrique, pilotée par un calculateur ou moyens de calcul 9 de façon à fournir une large gamme de débits et de pressions.

En sortie de l'enceinte 20, le générateur est relié à un premier circuit 3 dit circuit d'amenée du flux gazeux vers un masque respiratoire 4 ou un dispositif d'intubation (non-représenté) du patient.

Le générateur est commandé par des moyens 10 de commande sous la forme d'une carte électronique de commande reliée à une alimentation 24 et à des moyens de calcul 9 sous forme d'une carte électronique à microprocesseur pourvue d'un logiciel de pilotage et reliée à une interface 25 de commande avec écran et clavier.

Afin de piloter le fonctionnement du dispositif et de le faire fonctionner, les moyens de calcul sont reliés à des moyens 5, 6, 7, 8 de mesure de pression et/ou de mesure de débit du flux gazeux sous forme de capteurs.

Le capteur 6 est un capteur de pression relié à un premier circuit 3 circuit d'amenée du gaz au masque 4 ou dispositif d'intubation du patient.

Selon l'exemple le capteur 6 est disposé dans l'appareil 1 et relié au masque 4 ou au dispositif d'intubation au travers d'un tube 6a.

Le capteur 5 est un capteur de pression relié à un deuxième circuit 16 comportant une électrovanne proportionnelle 15 dont le fonctionnement sera décrit ultérieurement.

Le capteur 7 est un capteur de débit inspiré disposé sur le premier circuit, au niveau d'un lamineur 26 régulant le flux de gaz sortant du générateur.

Un capteur 8 de débit expiré, au niveau du troisième circuit 11 dit de retour patient complète les moyens de mesure.

Les moyens 9 de calcul commandent les organes tels que le générateur et l'électrovanne 15 proportionnelle en fonction de consignes entrées sur l'interface 25 en fonction de programmes de ventilation et en fonction des paramètres de pression et/ou de débit issus des capteurs.

Selon l'invention, les moyens de mesure 5, 6, 7, 8, les moyens 9 de calcul et les moyens 10 de commande de vitesse coopèrent en premier lieu pour asservir la vitesse de rotation de la turbine 2a en fonction des phases d'inspiration et d'expiration et en fonction de signaux de pression patient et/ou signaux de débit inspiré.

De façon à réguler le flux en sortie du générateur, le dispositif selon la figure 1 comprend un organe 12 passif de génération d'une perte de charge dans le circuit d'amenée sous la forme d'un clapet 12 anti-retour monté dans le premier circuit 3. Grâce à cet organe, la pression côté générateur est toujours légèrement supérieure à la pression dans le circuit 3 d'amenée côté masque ou dispositif d'intubation ce qui permet entre autre une meilleure régulation du fonctionnement du dispositif .

En aval de ce clapet 12 anti-retour, le circuit 3 comporte un clapet 13 passif d'admission d'air extérieur. Ce clapet permet de limiter les efforts d'inspirations du patient en respiration spontanée au travers de la machine lorsque celle-ci n'est pas en fonctionnement.

Côté masque 4 ou dispositif d'intubation après le circuit 3 d'amené du gaz, se trouve un troisième circuit 11 dit circuit d'expiration. Ce circuit d'expiration comporte une valve 14 d'expiration à membrane permettant de libérer un passage soit directement à l'extérieur soit au travers d'une tubulure 11a vers un lamineur 27 et une sortie d'air 28.

Sur la valve 14 se raccorde le deuxième circuit 16 pourvu de l'électrovanne 15 proportionnelle de type à trois voies. Cette électrovanne 15 reçoit sur une première voie une sortie de pression du générateur, comporte une deuxième voie à vide et est reliée par sa troisième voie à la valve 14.

L'électrovanne permet de piloter la valve 14 en appliquant une contre pression qui ferme la valve ou limite son ouverture tant que la pression d'expiration reste supérieure à la pression du deuxième circuit 16. Pour libérer la valve 14, l'électrovanne est pilotée de telle sorte que la pression du générateur soit bloquée, la deuxième voie à l'air libre étant mise en communication avec la troisième voie.

Ainsi un dispositif d'expiration piloté est construit autour de la valve 14 d'expiration pilotée par l'électrovanne 15 reliée au générateur de flux, cette électrovanne 15 permettant d'envoyer une partie du flux généré vers la valve d'expiration de façon à s'opposer à son ouverture.

L'électrovanne 15 proportionnelle est pilotée par des moyens de commande couplés aux moyens 9 de calcul et selon l'exemple disposés sur la carte recevant les moyens de calcul 9.

Ce dispositif permet ainsi en fonction du pilotage de la vitesse de la turbine 2a et du pilotage de la valve d'expiration au travers de l'électrovanne de fournir plusieurs modes de fonctionnement et notamment des modes de fonctionnement volumétriques. En effet plusieurs modes de fonctionnement sont recherchés et notamment des modes de fonctionnement à consignes de pression d'inspiration et d'expiration ou des modes pour lesquels un volume d'air dit volume cible est déterminé ou des modes mixtes.

Le fonctionnement de l'appareil est basé sur un système de pilotage auto-adaptatif en boucle fermée de la vitesse du générateur de flux. A ce générateur de flux est associé un dispositif d'expiration piloté, ce, sans organe de régulation du flux principal supplémentaire.

Notamment la détection d'une pente de montée en pression peut être traduite par un niveau d'accélération de la turbine 2a et la détection de la transition entre la phase d'inspiration et la phase d'expiration peut être traduite par une décélération de la turbine 2a.

Ce pilotage est réalisé à partir de signaux de pression ou de débit inspiré et selon des lois de pilotage, basées sur des coefficients proportionnels et intégraux, qui diffèrent suivant les modes de ventilation, les réglages de pression et ou débit et en particulier les phases du cycle respiratoire.

Le fonctionnement du dispositif et les procédés de ventilation associés seront expliqués par exemple dans le cas d'un mode pour lequel une pression d'inspiration Pi, une pression d'expiration Pe ainsi qu'une forme d'obtention de la pression d'inspiration sur la base d'un temps de montée en pression sont fixés. Le temps d'insufflation est ici dépendant d'un niveau de déclenchement d'expiration réglé sur la base de la mesure d'un chute de débit mesurée au niveau du capteur 7 d'inspiration, après l'obtention d'un débit maximum d'insufflation disponible pour le patient.

Un paramètre de limite pour le temps d'insufflation est le seuil minimum correspondant à la pente de pression mesurée un autre paramètre est un seuil maximum de sécurité pour le temps d'insufflation qui correspond au dernier temps inspiratoire réalisé ou au plus à 3s. D'autres paramètres de sécurité peuvent être pris en compte tel qu'une fréquence de sécurité.

Selon ce mode, l'objectif de la phase d'insufflation est l'établissement d'un niveau de pression Pi avec un temps de montée variable et un temps de maintien dépendant du comportement du débit associé.

L'objectif de la phase d'expiration est le maintien d'un niveau de pression Pe jusqu'au début de la phase d'inspiration suivante mais aussi de rincer suffisamment le circuit afin d'évacuer les gaz expirés résiduels.

Pour réaliser les cycles d'inspiration, les phases de montée en pression avec pentes de montée en pression sont réalisées par une accélération de la turbine 2a. La pression est contrôlée au niveau du capteur 6 de pression d'inspiration.

Pendant la phase d'insufflation correspondant à la phase d'inspiration, les moyens de calcul pilotent la valve 14 d'expiration, partie du troisième circuit 11 relié au masque 4 ou au dispositif d'intubation, au travers de l'électrovanne 15 proportionnelle et du deuxième circuit 16, en suiveur de pression.

La transition entre une phase d'inspiration et une phase d'expiration est réalisée elle par une décélération contrôlée de la turbine 2a et la valve 14 d'expiration, partie du troisième circuit 11 relié au masque 4 ou au dispositif d'intubation, est pilotée, au travers de l'électrovanne 15 proportionnelle et du deuxième circuit 16, en organe de régulation de pression du premier circuit 3.

Pendant la phase d'expiration, les moyens de calcul et les moyens de commande de vitesse de rotation du générateur adaptent la vitesse de la turbine 2a en fonction d'un seuil de pression expiratoire au delà d'une fuite régulée par la valve 14 d'expiration afin de créer le débit de rinçage du premier circuit.

Le système de clapet 12 anti-retour et de clapet 13 de respiration spontanée permet d'équilibrer la pression de rinçage qui est maintenue suffisamment faible pour limiter le phénomène de frein expiratoire sans être annulée pour éviter tout échauffement de la turbine 2a.

Dans le cas d'une ventilation pour laquelle un volume courant ainsi qu'une fréquence et un rapport de cyclage sont fixés, l'objectif de la phase d'insufflation est la distribution d'un volume courant Vt avec une forme de débit convenu et durant un temps fixé par les niveaux de fréquence et le rapport de cyclage réglés au travers de l'interface 25.

Dans ce cas, pendant l'insufflation, le circuit patient comportant le circuit 3, le masque 4 ou dispositif d'intubation est maintenu étanche, aux fuites parasites près par la fermeture de la valve 14 commandée par l'électrovanne 15. Il en résulte une montée en pression du circuit 3 et du système pulmonaire patient dépendant des caractéristiques du patient. En cas de dépassement d'objectif de pression maximale mesurée à l'aide du capteur de pression 6, il est possible de faire chuter la pression dans le circuit 16 de commande de la valve 14 par une commande de l'électrovanne 15 et de passer immédiatement en phase d'expiration.

Dans le cas d'un mode à volume cible, l'opérateur entre par les moyens d'interface une consigne de volume cible dans les moyens de calcul 9 qui, en fonction de ce paramètre et d'autres paramètres entrés par l'interface tels que fréquence de cycle minimale et/ou de sécurité, rapport de cyclage, seuil de pression haute, vont ajuster en permanence la pression d'insufflation entre un seuil de pression de base et un seuil de pression maximal afin de maintenir le volume courant inspiré par le patient au plus proche d'un volume cible prédéfini soit par exemple entre le volume cible Vt cible et Vt Cible + 20%.

Selon ce mode à volume cible, il est particulièrement nécessaire d'être à même d'ajuster précisément la pression par pas de pression entre les cycles Par exemple pour une pression de consigne de l'ordre de 20 mbar, les pas entre cycles sont définis entre 0,5 mbar et 2 mbar de façon à réagir immédiatement à la détection d'un déséquilibre sans avoir une réaction forte nuisible au confort du patient.

Cet ajustement de pression peut être réalisé avec un dispositif selon l'invention sans aucun dispositif régulateur en sortie du générateur mais en pilotant la vitesse de la turbine 2a du générateur 2 en fonction des mesures de pression du capteur 6 de pression patient et en faisant fonctionner le circuit d'expiration comportant la vanne d'expiration 14 en suiveur au travers de l'électrovanne 15 proportionnelle dans laquelle, dans cette configuration, le générateur de flux est relié au deuxième circuit, le clapet 12 anti-retour du premier circuit 3 permettant de conserver une pression légèrement supérieure dans le deuxième circuit par rapport au premier circuit.

Ainsi le dispositif permet de fournir un gaz respiratoire selon des cycles respiratoires comportant des phases d'inspiration et d'expiration, les moyens de mesure 5, 6, 7, 8 et les moyens 9 de calcul agissant sur les moyens 10 de commande de vitesse afin d'asservir la vitesse de rotation de la turbine 2a en fonction de consignes de pression et/ou de volume, de la détection de phases d'inspiration et d'expiration, de signaux de pression patient et/ou signaux de débit inspiré.

Avec le dispositif de l'invention la valve 14 d'expiration peut être pilotée en suiveur de pression lors de la phase d'inspiration et en organe de régulation durant la phase d'expiration.

Le dispositif selon l'invention permet ainsi de ventiler un patient en pression ou en volume avec un circuit patient doté d'un dispositif d'expiration piloté et une simple commande de vitesse de turbine.

## Revendications

1. - Dispositif (1) de fourniture de gaz respiratoire à un patient selon des cycles respiratoires, comportant un générateur (2) de flux gazeux muni d'une turbine (2a) à faible inertie et vitesse de consigne élevée, un premier circuit (3) dit circuit d'amenée du flux gazeux vers un masque respiratoire (4) ou un moyen d'intubation du patient, des moyens (5, 6, 7, 8) de mesure de pression et/ou de mesure de débit du flux gazeux, des moyens (9) de calcul de paramètres de pression et/ou de débit, des moyens (10) de commande de vitesse de rotation du générateur, et un système de pilotage de la vitesse du générateur comprenant les moyens de mesure (5, 6, 7, 8), les moyens (9) de calcul et les moyens (10) de commande de vitesse, lesdits moyens coopérant pour asservir la vitesse de rotation de la turbine (2a) en fonction des phases d'inspiration et d'expiration et en fonction de signaux de pression patient et/ou signaux de débit inspiré et **en ce caractérisé en ce qu'**il comporte:
- un deuxième circuit (16) de pilotage d'une valve d'expiration (14), partie d'un troisième circuit (11), par application d'une contre pression sur cette valve (14), ledit deuxième circuit (16) comportant une électrovanne proportionnelle (15) de type à trois voies comprenant une première voie reliée à une sortie de pression du générateur, une deuxième voie à l'air libre et une troisième voie reliée à la valve (14), des moyens de commande de ladite électrovanne (15) proportionnelle étant couplés aux moyens (9) de calcul,
et pour lequel l'électrovanne proportionnelle (15) et le deuxième circuit (16) sont agencés pour piloter la valve (14) d'expiration en organe de régulation de pression du premier circuit (3) lors de phases d'expiration.

2. - Dispositif selon la revendication 1 **caractérisé en ce que** les moyens de mesure de pression comprennent un capteur (6) de pression patient et/ou un capteur (5) de pression valve d'expiration.

3. - Dispositif selon la revendication 1 ou 2 **caractérisé en ce que** les moyens de mesure de débit comportent un capteur (7) de débit inspiré disposé à proximité de la sortie du générateur (2).

4. - Dispositif selon l'une quelconque des revendications précédentes **caractérisé en ce qu'**il comporte un organe (12) passif de génération d'une perte de charge dans le circuit (3) d'amenée.

5. - Dispositif selon la revendication 4 **caractérisé en ce que** cet organe (12) passif est un clapet anti-retour éventuellement associé à un clapet (13) passif d'admission d'air extérieur en aval du clapet anti-retour sur le circuit (3) d'amenée.

6. - Dispositif selon l'une quelconque des revendications précédentes **caractérisé en ce que** les moyens de mesure de débit comportent un capteur (8) de débit expiré au niveau d'un troisième circuit (11) dit de retour patient.

7. - Dispositif selon l'une quelconque des revendications précédentes **caractérisé en ce que** le système de pilotage du générateur est agencé pour réaliser des phases de montée en pression avec pentes de montée en pression au moyen d'une accélération de la turbine (2a) la fourniture du gaz respiratoire se faisant en pression ou en volume.

8. - Dispositif selon l'une quelconque des revendications précédentes **caractérisé en ce que** le système de pilotage du générateur est agencé pour réaliser la transition entre une phase d'inspiration et une phase d'expiration au moyen d'une décélération contrôlée de la turbine (2a).

9. - Dispositif selon l'une quelconque des revendications précédentes **caractérisé en ce que** le système de pilotage du générateur comporte des moyens pour ajuster en permanence la pression d'insufflation entre un seuil de pression de base et un seuil de pression maximal afin de maintenir le volume courant inspiré au plus proche d'un volume cible prédéfini.

10. - Dispositif selon l'une quelconque des revendications précédentes **caractérisé en ce que** les moyens (9) de calcul et les moyens (10) de commande de vitesse de rotation du générateur sont agencés pour adapter la vitesse de la turbine (2a) en fonction d'un seuil de pression expiratoire au delà d'une fuite régulée par la valve (14) d'expiration afin de créer un débit de rinçage du premier circuit (3) lors de phases d'expiration.

11. - Dispositif selon l'une quelconque des revendications précédentes **caractérisé en ce que** l'électrovanne (15) proportionnelle et le deuxième circuit (16) sont agencés pour piloter la valve (14) d'expiration en suiveur de pression lors de phases d'insufflation correspondant aux phases d'inspiration.

## Claims

1. A device (1) for supplying respiratory gas to a patient in respiratory cycles, comprising a gas-flow generator (2) provided with a low-inertia turbine (2a), with a high set speed, a first circuit (3) referred to as the circuit for feeding the gas flow to a respiratory mask (4) or means for intubating the patient, means (5, 6, 7, 8) for measuring pressure and/or measuring the gas flow rate, means (9) for calculating pressure and/or flow rate parameters, and means (10) for controlling the rotational speed of the generator, and a system for controlling the speed of the generator comprising the measuring means (5, 6, 7, 8), the computing means (9) and the speed control means (10), said means cooperating in order to control the speed of rotation of the turbine (2a) according to the inhalation and exhalation phases and as a function of patient pressure signals and/or inhaled rate signals;
**characterised in that** it comprises:
- a second circuit (16) for controlling an exhalation valve (14), part of a third circuit (11), by application of a counter-pressure on this valve (14), said second circuit (16) comprising a proportional solenoid valve (15) of three-way type comprising a first way connected to a pressure outlet of the generator, a second way to open air and third way connected to the valve (14), means for controlling said proportional solenoid valve (15) being coupled to the computing means (9),
and for which the proportional solenoid valve (15) and the second circuit (16) are arranged so as to control the exhalation valve (14) as a pressure regulation member for the first circuit (3) during exhalation phases.

2. A device according to claim 1, **characterised in that** the pressure measuring means comprises a patient pressure sensor (6) and/or an exhalation valve pressure sensor (5).

3. A device according to claim 1 or 2, **characterised in that** the rate measurement means comprises an inhaled rate sensor (7) disposed close to the outlet of the generator (2).

4. A device according to any one of the preceding claims, **characterised in that** it comprises a passive member (12) for generating a pressure drop in the feed circuit (3).

5. A device according to claim 4, **characterised in that** this passive member (12) is a non-return valve possibly associated with a passive valve (13) admitting external air downstream of the non-return valve on the feed circuit (3).

6. A device according to any one of the preceding claims, **characterised in that** the rate measurement means comprises an exhaled rate sensor (8) at a third so-called patient-return circuit (11).

7. A device according to any one of the preceding claims, **characterised in that** the system for controlling the generator is arranged to produce pressure-rise phases with pressure-rise slopes by means of an acceleration of the turbine (2a), the supply of respiratory gas taking place in terms of pressure or volume.

8. A device according to any one of the preceding claims, **characterised in that** the control system of the generator is arranged to produce the transition between an inhalation phase and an exhalation phase by means of a controlled deceleration of the turbine (2a).

9. A device according to any one of the preceding claims, **characterised in that** the control system of the generator comprises means for continuously adjusting the insufflation pressure between a base pressure threshold and a maximum pressure threshold in order to maintain the current volume inhaled as close as possible to a predefined target volume.

10. A device according to any one of the preceding claims, **characterised in that** the computing means (9) and the means (10) for controlling the rotation speed of the generator are arranged to adapt the speed of the turbine (2a) according to an exhalation pressure threshold beyond a leakage regulated by the exhalation valve (14) in order to create a rinsing rate for the first circuit (3) during exhalation phases.

11. A device according to any one of the preceding claims, **characterised in that** the proportional solenoid valve (15) and the second circuit (16) are arranged so as to control the exhalation valve (14) as a pressure follower during insufflation phases corresponding to the inhalation phases.

## Patentansprüche

1. Vorrichtung (1) zur Lieferung von Atmungs-Gas an einen Patienten gemäß der Atmungszyklen welche umfasst : einen Generator (2) eines Gas-Flusses, welcher mit einer Turbine (2a) mit niedriger Trägheit und hoher Sollgeschwindigkeit ausgestattet ist, einen ersten Kreislauf (3), genannt Zuführungs-Kreislauf des Gas-Flusses zu einer Atmungs-Maske (4) oder einem Mittel zum Intubieren des Patienten, Mittel (5,6,7,8) zur Messung des Drucks und/oder zur Messung des Durchsatzes des Gas-Flusses, Mittel (9) zur Berechnung von Druckparametern und/oder des Durchflussses des Gas-Flusses, Mittel (10) zur Steuerung der Dreh-Geschwindigkeit des Generators und ein System zur Steuerung der Geschwindigkeit des Generators, welches umfasst : die Mittel zur Messung (5,6,7,8), die Mittel (9) zur Berechnung und die Mittel (10) zur Steuerung der Geschwindigkeit, wobei die besagten Mittel zusammenarbeiten, um die Dreh-Geschwindigkeit der Turbine (2a) den Einatmungs- und Ausatmungs-Phasen und den Drucksignalen und/oder eingeatmeten Durchsatz-Signalen zu unterwerfen, **gekennzeichnet dadurch, dass** sie umfasst
• einen zweiten Kreislauf (16) zur Steuerung eines Ausatmungs-Ventils (14), welches Teil eines dritten Kreislaufs (11) ist, durch Anwendung eines Gegendrucks auf diesem Ventil (14), wobei der besagte zweite Kreislauf (16) ein proportionales Drei-Wege-Magnetventil (15) umfasst, welches einen ersten Weg aufweist, der mit dem Druck-Ausgang des Generators verbunden ist, einen zweiten Weg zur Umgebung und einen dritten Weg, der mit dem Ventil (14) verbunden ist, wobei die Mittel zur Steuerung des besagten proportionalen Magnetventils (15) mit dem Mittel (9) zur Berechnung verbunden sind,
und für welche das proportionale Magnetventil (15) und der zweite Kreislauf (16) eingerichtet sind, um das Ausatmungs-Ventil (14) als Regulierungs-Organ des Drucks des ersten Kreislaufs (3) während der Ausatmungs-Phase zu steuern.

2. Vorrichtung gemäß Anspruch 1, **gekennzeichnet dadurch, dass** die Mittel zur Messung des Drucks einen Sensor (6) des Patienten-Drucks und/oder einen Sensor (5) des Drucks des Ausatmungs-Ventils umfassen.

3. Vorrichtung gemäß der Ansprüche 1 oder 2 **gekennzeichnet dadurch, dass** die Mittel zur Messung des Durchsatzes einen Sensor (7) des eingeatmeten Durchsatzes umfassen, welcher in der Nähe des Ausgangs des Generators (2) angebracht ist.

4. Vorrichtung gemäß einem beliebigen der vorangegangen Ansprüche **gekennzeichnet dadurch, dass** sie ein passives Element (12) zur Generierung eines Ladungs-Verlustes im Zuführungs-Kreislauf (3) umfasst.

5. Vorrichtung gemäß des Anspruches 4 **gekennzeichnet dadurch, dass** dieses passive Element (12) eine Anti-Rückfluss-Klappe ist, welche möglicherweise mit einer passiven Klappe (13) zur Zuführung von externer Luft flussabwärts von der Anti-Rückfluss-Klappe in dem Zuführungs-Kreislauf (3) assoziiert ist.

6. Vorrichtung gemäß einer beliebigen der vorrangegangenen Ansprüche **gekennzeichnet dadurch, dass** die Mittel zur Messung des Durchsatzes einen Sensor (8) des ausgeatmeten Durchsatzes in Höhe des dritten Kreislaufs (11), genannt Rückführung vom Patienten, umfassen.

7. Vorrichtung gemäß einem beliebigen der vorangegangenen Ansprüche, **gekennzeichnet dadurch, dass** das System zur Steuerung des Generators eingerichtet ist, um Phasen der Druck-Steigerung mit einer Druck-Steigerungs-Steigung mittels Beschleunigung der Turbine (2a) zu realisieren, wobei die Lieferung des Atmungs-Gases nach Druck oder Volumen erfolgt.

8. Vorrichtung gemäß einem beliebigen der vorangegangenen Ansprüche, **gekennzeichnet dadurch, dass** das System zur Steuerung des Generators eingerichtet ist, um den Übergang zwischen einer Einatmungsphase und einer Ausatmungsphase anhand einer kontrollierten Abbremsung der Turbine (2a) zu realisieren.

9. Vorrichtung gemäß einem beliebigen der vorangegangenen Ansprüche, **gekennzeichnet dadurch, dass** das System zur Steuerung des Generators Mittel umfasst, um ständig den Einblase-Druck zwischen einer Schwelle eines Basis-Drucks und einer Schwelle eines Maximal-Drucks zu adjustieren, um das laufende eingeatmete Volumen möglichst nah an einem vordefinierten Ziel-Volumen zu halten.

10. Vorrichtung gemäß einem beliebigen der vorangegangenen Ansprüche, **gekennzeichnet dadurch, dass** die Mittel (9) zur Berechnung und die Mittel (10) zur Steuerung der Dreh-Geschwindigkeit des Generators eingerichtet sind, um die Geschwindigkeit der Turbine (2a) in Abhängigkeit von einer Schwelle des Ausatmungs-Drucks über ein reguliertes Entweichen durch das Ausatmungs-Ventil (14) anzupassen, um einen Spülungs-Fluss des ersten Kreislaufs (3) während der Ausatmungs-Phase zu erzeugen.

11. Vorrichtung gemäß einem beliebigen der vorangegangenen Ansprüche, **gekennzeichnet dadurch, dass** das proportionale Magnetventil (15) und der zweite Kreislauf (16) eingerichtet sind, um das Ausatmungs-Ventil (14) als Druck-Begleiter während der Einblase-Phase, welche mit der Einatmungsphase korrespondiert, zu steuern.
